# EUROPEAN PATENT SPECIFICATION

(11) **EP 4 125 916 B1**
(45) Date of publication and mention of the grant of the patent: **17.04.2024**
(21) Application number: 21718411.8
(22) Date of filing: 22.03.2021
(51) Int. Cl.: A61K 31/5365, A61P 35/00, A61P 35/02

(54) **METHOD FOR TREATING IDH1 INHIBITOR-RESISTANT SUBJECTS**
VERFAHREN ZUR BEHANDLUNG VON IDH1-INHIBITORRESISTENTEN PATIENTEN
PROCÉDÉ DE TRAITEMENT DE SUJETS RÉSISTANTS À L'INHIBITEUR DE L'IDH1

(30) Priority: 23.03.2020 US 202062993135 P; 14.05.2020 US 202063024761 P; 22.02.2021 US 202163151905 P
(43) Date of publication of application: 08.02.2023
(73) Proprietor: ELI LILLY AND COMPANY, Indianapolis, IN 46285 (US)
(72) Inventor: BROOKS, Nathan, Arthur, Indianapolis, IN 46206-6288 (US); GILMOUR, Raymond, Indianapolis, IN 46206-6288 (US)
(74) Representative: J A Kemp LLP
(86) International application number: PCT/US2021/023452
(87) International publication number: WO 2021/194953

(56) References cited:
- WO-A1-2018/111707
- SUNG CHOE, ET AL.: "Molecular mechanisms mediating relapse following ivosidenib monotherapy in patients with IDH1-mutant relapsed or refractory acute myeloid leukemia", Presentation 545, 61st American Society of Hematology Annual Meeting , November 2019 (2019-11), pages 1-16, XP055816208, Retrieved from the Internet: URL:https://www.servier.us/sites/default/f iles/2021-04/ASH19_IVO_relapse.pdf [retrieved on 2021-06-21] cited in the application

## Description

The present invention relates to the treatment of cancer in subjects with a mutant isocitrate dehydrogenase 1 (IDH1) inhibitor disclosed herein.

IDH1 is an enzyme that catalyzes the conversion of isocitrate to α-ketoglutarate (α -KG) and reduces nicotinamide adenine dinucleotide phosphate (NADP⁺) to NADPH (Megias-Vericat J, et al., Blood Lymph. Cancer: Targets and Therapy 2019; 9: 19-32).

Neomorphic (*de* novo) mutations in IDH1, *e.g.,* at IDH1 amino acid residue R132, contribute to tumorigenesis in several types of cancer, including solid tumors and hematologic malignancies (Badur MG, et al., Cell Reports 2018; 25: 1680). IDH1 mutations can result in high levels of 2-hydroxyglutarate (2-HG), which inhibits cellular differentiation, and inhibitors of mutant IDH1 can reduce 2-HG levels, which promotes cellular differentiation (Molenaar RJ, et al., Oncogene 2018; 37: 1949-1960).

For example, acute myeloid leukemia (AML) is characterized by a diverse spectrum of mutated genes and a multi-clonal genomic architecture comprising preleukemic and leukemic clones that evolve dynamically over time and under the selective pressure of therapy (Bloomfield CD, et al., Blood Revs. 2018: 32: 416-425).

Induction chemotherapy with cytarabine and an anthracycline ("7 + 3") has been the standard of care for more than 4 decades for subjects with newly diagnosed AML. In recent years, five drugs have been approved by the U.S. Food and Drug Administration for treating AML: midostaurin, enasidenib, CPX-351, gemtuzumab ozogamicin (Bloomfield CD, et al., Blood Revs. 2018; 32: 416-425), and ivosidenib (Megias-Vericat J, et al., Blood Lymph. Cancer: Targets and Therapy 2019; 9: 19-32).

Approximately 60% to 70% of adults with AML can be expected to attain complete remission (CR) status following appropriate induction therapy, and more than 25% of adults with AML (about 45% of those who attain CR) can be expected to survive 3 or more years and may be cured.

However, IDH1 resistance mutations are observed in 7-14% of AML subjects, and the associated high 2-HG level can result in an epigenetic hyper-methylation phenotype and a block in differentiation, resulting in leukemogenesis (Megias-Vericat J, et al., Blood Lymph. Cancer: Targets and Therapy 2019; 9: 19-3). In addition, mutations in the Flt3 kinase are observed in approximately one third of AML subjects (Lee HJ, et al., Oncotarget 2018; 9: 924-936).

So called "secondary" IDH1 mutations, as defined herein, may contribute to relapse after treatment with a mutant IDH1 inhibitor. For example, to date, six post-ivosidenib treatment secondary IDH1 mutations have been reported: R119P, G131A, D279N, S280F, G289D or H315D (Choe S, et al., "Molecular mechanisms mediating relates following ivosidenib monotherapy in subjects with IDH1-mutant relapsed or refractory acute myeloid leukemia," 61st Am. Soc. Hematol. (ASH) Annual Meeting poster, Dec. 7-10, 2019, Orlando, FL, USA; Choe, S., et al., Blood Adv. 2020, 4(9): 1894-1905).

Thus, there remains a need for alternative mutant IDH1-related cancer therapies, particularly for subjects in whom secondary IDH1 mutations occur after initial mutant IDH1 inhibitor therapy.

Certain mutant IDH1 inhibitors are disclosed in WO 2018/111707 A1, including a compound defined herein as "Compound A," which is a covalent inhibitor of mutant IDH1 that modifies a single cysteine (Cys269) in an allosteric binding pocket, rapidly inactivates the enzyme, and selectively inhibits 2-HG production, without affecting α-KG levels (WO 2018/111707 A1).

The present invention provides a compound of the Formula I: 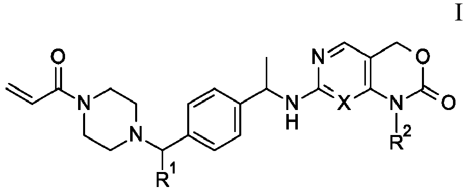 wherein:
R¹ is -CH₂CH(CH₃)₂, -CH₂CH₃, -CH₂CH₂OCH₃, or -CH₂-cyclopropyl;
R² is -CH₃ or -CH₂CH₃; and
X is N or CH;
or a pharmaceutically acceptable salt thereof;
for use in treating cancer in a human subject having an IDH1 R132 mutation and one or more secondary IDH1 mutations.

In one embodiment of the compound of Formula I, X is N, or a pharmaceutically acceptable salt thereof. In another embodiment, X is N, R¹ is -CH₂-cyclopropyl, and R² is -CH₂CH₃, or a pharmaceutically acceptable salt thereof. In another embodiment, X is N, R¹ is -CH₂-cyclopropyl, and R² is -CH₂CH₃.

In another embodiment, the compound of Formula I is:
7-[[(1S)-1-[4-[(1R)-2-Cyclopropyl-1-(4-prop-2-enoylpiperazin-1-yl)ethyl]phenyl]ethyl]amino]-1-ethyl-4H-pyrimido[4,5-d][1,3]oxazin-2-one;
7-[[(1S)-1-[4-[(1S)-2-cyclopropyl-1-(4-prop-2-enoylpiperazin-1-yl)ethyl]phenyl]ethyl]amino]-1-ethyl-4H-pyrimido[4,5-d][1,3]oxazin-2-one; or
1-Ethyl-7-[[(1S)-1-[4-[1-(4-prop-2-enoylpiperazin-1-yl)propyl]phenyl]ethyl]amino]-4H-pyrimido[4,5-d][1,3]oxazin-2-one;
or a pharmaceutically acceptable salt of any one thereof.

In another embodiment, the compound of Formula I is 7-[[(1S)-1-[4-[(1S)-2-cyclopropyl-1-(4-prop-2-enoylpiperazin-1-yl)ethyl]phenyl]ethyl]amino]-1-ethyl-4H-pyrimido[4,5-d][1,3]oxazin-2-one.

In another embodiment, the compound of Formula I is: 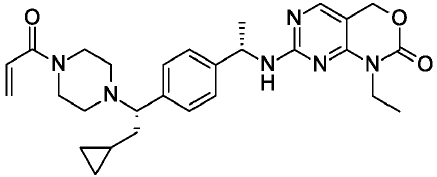 (referred to herein as "Compound A"), or a pharmaceutically acceptable salt thereof. In another embodiment, the compound is Compound A.

In one embodiment of the invention, the R132 mutation is R132H In another embodiment, the IDH1 mutation is R132C. In another embodiment, the IDH1 mutation is R132G. In another embodiment, the IDH1 mutation is R132L. In another embodiment, the IDH1 mutation is R132S.

In one embodiment of the invention, the one or more secondary IDH1 mutations is one or more of R119P, G131A, D279N, S280F, G289D or H315D. In another embodiment, the secondary IDH1 mutation is two or more of R119P, G131A, D279N, S280F, G289D or H315D.

In another embodiment, the subject is identified as having an R132 IDH1 mutation. In another embodiment, the subject is identified as having an R132 IDH1 mutation in tissue.

In another embodiment, the subject is identified as having one or more secondary IDH1 mutations.

In another embodiment, the cancer is a hematologic malignancy, and the subject is identified as having an R132 IDH1 mutation in blood, bone marrow, lymph node or lymphatic fluid. In another embodiment, the subject is identified as having an R132 IDH1 mutation in blood cells, bone marrow cells, or blood cells, or lymph node cells, or lymphatic fluid cells. In another embodiment, the subject is identified as having one or more secondary IDH1 mutations.

In another embodiment, the cancer is a solid tumor cancer, and the subject is identified as having an R132 IDH1 mutation in solid tumor tissue. In another embodiment, the solid tumor tissue is cholangiocarcinoma tissue. In another embodiment, the subject is identified as having an R132 IDH1 mutation in solid tumor tissue cells. In another embodiment, the subject is identified as having one or more secondary IDH1 mutations.

In one embodiment of the method of the invention, the cancer is a solid tumor. In another embodiment, the solid tumor is cholangiocarcinoma, head & neck cancer, chondrosarcoma, hepatocellular carcinoma, melanoma, pancreatic cancer, astrocytoma, oligodendroglioma, glioma, glioblastoma, bladder carcinoma, colorectal cancer, lung cancer, or sinonasal undifferentiated carcinoma. In another embodiment, the lung cancer is non-small cell lung cancer. In another embodiment, the solid tumor is cholangiocarcinoma.

In another embodiment, the cancer is a hematologic malignancy.
In another embodiment, the hematologic malignancy is acute myeloid leukemia, myelodysplastic syndrome myeloproliferative neoplasm, angioimmunoblastic T-cell lymphoma, T-cell acute lymphoblastic leukemia, polycythemia vera, essential thrombocythemia, primary myelofibrosis or chronic myelogenous leukemia. In another embodiment, the hematologic malignancy is acute myeloid leukemia.

In another embodiment, the subject had been treated with a mutant IDH1 inhibitor other than a compound of Formula I. In another embodiment, the mutant IDH1 inhibitor other than a compound of Formula I is vorasidenib, BAY-1436032, AGI-5198, IDH305, or ivosidenib. In another embodiment, the mutant IDH1 inhibitor other than a compound of Formula I is ivosidenib. In another embodiment, the subject had been treated with a mutant IDH1 inhibitor other than a compound of Formula I prior to treatment with a compound of Formula I.

In another embodiment of the present invention, the cancer is relapsed cancer. In another embodiment, the relapsed cancer is a solid tumor cancer. In another embodiment, the relapsed solid tumor cancer is cholangiocarcinoma. In another embodiment, the relapsed cancer is hematologic malignancy. In another embodiment, the relapsed hematologic malignancy is relapsed AML.

In another embodiment of the present invention, the cancer is refractory cancer. In another embodiment, the refractory cancer is a solid tumor cancer. In another embodiment, the refractory solid tumor cancer is cholangiocarcinoma. In another embodiment, the refractory cancer is hematologic malignancy. In another embodiment, the refractory hematologic malignancy is refractory AML.

In another embodiment of the present invention, the cancer is advanced cancer. In another embodiment, the advanced cancer is an advanced solid tumor cancer. In another embodiment, the advanced solid tumor cancer is cholangiocarcinoma. In another embodiment, the advanced cancer is an advanced hematologic malignancy. In another embodiment, the advanced hematologic malignancy is advanced AML.

In another embodiment, the AML is acute promyelocytic leukemia.

As used above, and throughout the description of the invention, the following terms, unless otherwise indicated, shall be understood to have the following meanings:

The term "hematologic tissue" refers to blood, bone marrow, spleen, lymph node, or lymphatic fluid.

The term "solid tumor tissue" refers to tissue that is not hematologic tissue. Nonlimiting examples of solid tissue are cholangial tissue, pancreatic tissue, head tissue, neck tissue, hepatic tissue, skin tissue, astrocytomal tissue, oligodendroglial tissue, glial tissue, brain tissue, bladder tissue, colorectal tissue, lung tissue, and sinonasal undifferentiated carcinoma.

The term "solid tumor cancer" means that the cancer originated in a tissue that is not blood, bone marrow, lymph node or lymphatic fluid.

The term "hematologic malignancy" relates to cancer that in the blood, the bone marrow, the lymph node or the lymphatic fluid.

The term "advanced hematological malignancy" refers to malignancy that has spread to lymph nodes or to other tissues outside of the blood or the bone marrow.

The term "cancer subject" means a subject who has been diagnosed with cancer.

The term "refractory cancer" refers to cancer that has been treated, but the human cancer subject did not respond to treatment.

The term "relapsed cancer" means that the human cancer subject responded to treatment for a period of time, but that the cancer has reoccurred.

The term "advanced cancer" refers to cancer that has spread to lymph nodes or to other tissues outside of the cancer's point of origin. For example, advanced acute myeloid leukemia is acute myeloid leukemia that has spread to a tissue outside of the blood or the bone marrow.

The term "solid tumor subject" means a subject who has been diagnosed with a solid tumor cancer. In one embodiment, the solid tumor cancer is cholangiocarcinoma.

The term "hematologic malignancy subject" means a subject who has been diagnosed with a hematologic malignancy. In one embodiment, the hematologic malignancy subject is an AML subject. The term "AML subject" means a subject who has been diagnosed with AML. Methods for diagnosing AML are known to those of ordinary skill in the art, *e.g.,* in Dohner H, et al., Blood 2017; 129: 424-447.

The terms "acute myeloid leukemia," "acute myelogenous leukemia," and "acute nonlymphocytic leukemia" are synonymous.

"Responsiveness to hematologic malignancy (*e*.*g*., AML) treatment" includes improvement in overall survival, partial response, long-term stable disease, or improvement in long-term survival characterized as complete remission (determined by less than 5% myeloblasts in bone marrow, the absence of circulating blasts, hematologic recovery (as evidenced by a peripheral blood absolute neutrophil count greater than 1,000 cells/µL and a platelet count greater than 100,000/µL, without the need for red blood cell transfusion, and the absence of extramedullary disease) (Bloomfield CD, et al., Blood Revs. 2018; 32: 416-425).

The term "IDH1 R132 mutation" refers to an IDH1 mutation at amino acid residue 132 in a subject's IDH1 gene, as determined, *e*.*g*., in the subject's nucleic acid (*e.g.,* DNA). As used herein, an "IDH1 R132 mutation" is not a "secondary IDH1 mutation."

The term "secondary IDH1 mutation" refers to an IDH1 mutation that occurs in the IDH1 enzyme in a human subject after treatment with a mutant IDH1 inhibitor other than a compound of Formula I herein. In one embodiment of the method of the invention, the one or more secondary IDH1 mutations is one or more of R119P, G131A, D279N, S280F, G289D or H315D in IDH1. However, other secondary IDH1 mutations may be reported in the future. As used herein, a "secondary IDH1 mutation" is not an "IDH1 R132 mutation."

The term "mutant IDH1 inhibitor" refers to a compound that inhibits the enzyme activity of and/or the production of 2-HG by a mutant IDH1 enzyme. Methods for assaying mutant IDH1 enzyme activity are known to those of ordinary skill in the art, *e.g.,* in WO 2018/111707 A1. In the term "mutant IDH1 inhibitor, the word "mutant" refers to the IDH1 gene, not the inhibitor.

The term "identified as having an IDH1 R132 mutation" means that nucleic acid (*e.g.,* DNA) from a human subject's tissue or cells has been analyzed to determine if the human subject has an IDH1 R132 mutation. In one embodiment, one or more of the human subject's blood cells, bone marrow cells, lymph node, lymph node cells, lymphatic fluid or lymphatic fluid cells has been analyzed for an IDH1 R132 mutation. In another embodiment, the human subject's solid tissue has been analyzed for an IDH1 R132 mutation.

In the method of the present invention, the party who identifies the human subject as having an IDH1 R132 mutation can be different than the party that administers the compound. In one embodiment, the party who identifies the human subject as having an IDH1 R132 mutation is different than the party that administers the compound.

The term "identified as having one or more secondary IDH1 mutation(s)" means that nucleic acid (*e*.*g*., DNA) from one or more of the human subject's blood cells, bone marrow cells, lymph node, lymph node cells, lymphatic fluid or lymphatic fluid cells has been analyzed to determine if a human subject has one or more secondary IDH1 mutation(s).

Analytical methods for identifying genetic mutations are known to those of ordinary skill in the art (Clark, O., et al., Clin. Cancer. Res. 2016; 22: 1837-42), including, but not limited to, karyotyping (Guller JL, et al., J. Mol. Diagn. 2010; 12: 3-16), fluorescence *in situ* hybridization (Yeung DT, et al., Pathology 2011; 43: 566-579), Sanger sequencing (Lutha, R et al., Haematologica 2014; 99: 465-473), metabolic profiling (Miyata S, et al., Scientific Reports 2019; 9: 9787), polymerase chain reaction (Ziai, JM and AJ Siddon, Am. J. Clin. Pathol 2015; 144: 539-554), and next-generation sequencing (*e.g.,* whole transcriptome sequencing) (Lutha, R et al., Haematologica 2014; 99: 465-473; Wang H-Y, et al., J. Exp. Clin. Cancer Res. 2016; 35: 86).

The terms "treatment," "treat," "treating," and the like, are meant to include slowing, stopping, or reversing the progression of cancer. These terms also include alleviating, ameliorating, attenuating, eliminating, or reducing one or more symptoms of a disorder or condition, even if the cancer is not actually eliminated and even if progression of the cancer is not itself slowed, stopped or reversed. "Therapeutically effective amount" means the amount of a compound, or pharmaceutically acceptable salt thereof, administered to the subject that will elicit the biological or medical response of or desired therapeutic effect on a subject. A therapeutically effective amount can be readily determined by the attending clinician, as one skilled in the art, by the use of known techniques and by observing results obtained under analogous circumstances. In determining the effective amount for a subject, a number of factors are considered by the attending clinician, including, but not limited to: size, age, and general health of the subject; the specific disease or disorder involved; the degree of or involvement or the severity of the disease or disorder; the response of the individual subject; the particular compound administered; the mode of administration; the bioavailability characteristics of the preparation administered; the dose regimen selected; the use of concomitant medication; and other relevant circumstances.

A compound of Formula I herein can optionally be formulated as a pharmaceutical composition administered by any route which makes the compound bioavailable, including oral, intravenous, and transdermal routes. It is preferred that such compositions are formulated for oral administration. Such pharmaceutical compositions and processes for preparing same are well known in the art. (See, e.g., Remington: The Science and Practice of Pharmacy (D.B. Troy, Editor, 21st Edition, Lippincott, Williams & Wilkins, 2006).

A "pharmaceutically acceptable carrier, diluent, or excipient" is a medium generally accepted in the art for the delivery of biologically active agents to mammals, e.g., humans.

It will be understood by one of ordinary skill in the art that compounds administered in the method of the invention are capable of forming salts. The compounds react with any of a number of inorganic and organic acids to form pharmaceutically acceptable acid addition salts. Such pharmaceutically acceptable acid addition salts and common methodology for preparing them are well known in the art. *See, e.g.,* P. Stahl, et al., HANDBOOK OF PHARMACEUTICAL SALTS: PROPERTIES, SELECTION AND USE, (VCHA/Wiley-VCH, 2008).

"Pharmaceutically acceptable salts" or "a pharmaceutically acceptable salt" refers to the relatively non-toxic, inorganic and organic salt or salts of the compounds of the present invention (S.M. Berge, et al., "Pharmaceutical Salts", Journal of Pharmaceutical Sciences, Vol 66, No. 1, January 1977).

### Materials, Methods and Results

**Compounds and Formulation.** Ivosidenib and Compound A are prepared as a 20 mM stock in 100% DMSO (dimethyl sulfoxide) (Sigma, D2438) and diluted serially in 100% DMSO to achieve the desired concentrations. DMSO preps are further diluted with cell culture media prior to addition in the assay.

**Cell lines.** U-87 MG cells (ATCC, HTB-14) are cultured and assayed in MEM (Gibco, 11095) with 2 mM GlutaMAX (Gibco, 35050), 1 mM Pyruvate (Gibco, 11360), 0.1 mM NEAA ((Non-essential amino acid) Gibco, 11140) and 10% dialyzed FBS (Fetal bovine serum) (Gibco, 26400). Ba/F3 cells (DSMZ, ACC 300) are cultured and assayed in RPMI 1640 (Gibco, 22400) with 10% heat inactivated FBS (Gibco, 10082-147) and 10 ng/ml mouse IL3 (R&D systems, 403-MI,-025).

**Cell-based inhibition assays.** Cell-based assays are performed by measuring 2-HG in either U-87 MG cells or Ba/F3 cells in which IDH mutations are expressed.

DNA constructs encoding IDH1 mutations are introduced into U-87 MG cells using transfection (Promega FuGENE HD, E2311) or lentiviral transduction, and the IDH-mutant expressing cell lines are selected using blasticidin (5 µg/ml) or puromycin (1 µg/ml). For compound treatment in U-87 MG cells, 20,000-50,000 cells per well are plated in 96 well cell culture plates (Falcon, 353377) 2hrs prior to treatment. Cells are treated with serial dilutions of compound A in standard growth media. Plates are incubated in a mammalian cell culture incubator (humidified, 37°C, 5%CO₂) for 16-72 hrs. Following the incubation period, the media is aspirated and cell lysates are prepared either by addition of 30 µL/well lysis buffer (25 mM Tris-HCl pH7.5), 150 mM NaCl, 1 mM EDTA, 1 mM EGTA, 1% Triton-X 100, 2X Halt protease + phosphatase inhibitor (Pierce, 78441) (for derivatization LC-MS method) or by addition of 100 µL 80% methanol/20% water containing LC-MS internal standards (1 µM ¹³C₄ α-KG/¹³C₅ 2-HG) per well (for ion pairing LC-MS method). 96-well sample plates are then sealed, shaken at 450 rpm for 10 min, and then placed at -20°C and stored until LC-MS analysis.

Ba/F3 cells are transfected with DNA constructs encoding IDH1R132H-myc, IDH1R132H_S280F-myc, or IDH1R132C_S280F-myc constructs using NEON Transfection system (Life Technologies, MPK10025) and isolated using Puromycin (2 µg/mL) or Blasticidin (10 µg/mL). Stably transfected lines are used for inhibitor assays. 15,000 Ba/F3 cells per well are plated in 96 well cell culture plates (Falcon, 353377) 2 hours prior to treatment. Cells are treated with serial dilutions of the desired compounds in standard growth media. Plates are incubated in a mammalian cell culture incubator (humidified, 37 °C, 5% CO₂) for the desired time (72 or 96 hours). Following the incubation period, conditioned media from each well is collected for 2-HG analysis by LC-MS.

**LC-MS metabolite analysis of conditioned media and cell lysates.** The effect of inhibitors on the concentrations of 2-HG are determined by liquid chromatography-mass spectrometry (LC-MS) analysis of cell lysates or conditioned media using either a derivatization method or an ion-pairing method as described below.

For the derivatization LC-MS method, calibration curves are prepared by spiking 2-HG and α-KG into cell culture media and cell lysis buffer respectively. The method utilizes derivatization with O-benzylhydroxylamine prior to analysis by LC-MS. 10 µL of each standard or sample (media or cell extract) is placed into a deep-well 96-well plate and combined with 100 µL of internal standard solution containing 10 µM d5-3-hydroxyglutarate and 10 µM d6-α-KG. 50 µL of 1M O-benzylhydroxylamine in pyridine buffer (8.6% pyridine, pH 5) and 50 µL of 1 M N-(3-dimethylaminopropyl)-N-ethylcarbodiimide hydrochloride (EDC) in pyridine buffer is added to each sample. The derivatization reaction proceeds at room temperature for one hour. Using a Beckman Biomek FX liquid handler, 300 µL of ethyl acetate is added to each sample. Plates are sealed and vortexed for 5 minutes, followed by centrifugation for 5 minutes at 4000 rpm in Eppendorf 5810R centrifuge. 220 µL of the upper layer is transferred to a new 96-well plate. Samples are dried under heated nitrogen at 50 °C and reconstituted with 100 µL of methanol/water (1:1). 1 µL of derivatized sample is injected onto an LC-MS system consisting of a Shimadzu Prominence 20A HPLC system and a Thermo Quantum Ultra^{™} triple quadrupole mass spectrometer. Analytes are separated on a Water XBridge^{™} C18 column (2.1 x 50 mm, 3.5 µm) with a flow rate of 0.6 mL/minute. Mobile phase A is 0.1% formic acid in water and mobile phase B is methanol. The gradient profile is: 0 minutes, 5% B; 2 minutes, 100% B; 4.00 minutes, 100% B; 4.1 minutes, 5% B; 5.50 minutes, stop. The mass spectrometer utilizes a HESI-II probe operated in positive ion selected reaction monitoring mode. Calibration curves are constructed by plotting analyte concentrations vs. analyte/internal standard peak area ratios and performing a quadratic fit of the data using a 1/concentration weighting with Xcalibur^{™} software. Analyte concentrations for the unknowns are back calculated from the calibration curves.

For the ion-pairing LC-MS method, calibration curves are prepared by spiking 2-HG and α-KG into 80% methanol/20% water containing LC-MS internal standards (1 µM ¹³C₄ α-KG/¹³C₅ 2-HG). The quantitation of 2-HG and α-KG is accomplished using an AB Sciex 6500 mass spectrometer with an ESI probe and interfaced with an UHPLC system in the negative multiple-reaction monitoring (MRM) mode. The UHPLC system consists of an Agilent 1290 binary pump, thermostatted column compartment (TCC), and sampler. The injection volume is 1 µL for cell culture extracts. The extracts are chromatographically resolved using a Hypercarb column, 2.1x20 mm, 5.0 mm Javelin HTS (Thermo Scientific, PN: 35005-022135). Mobile phase A is water/10mM tributylamine/15mM acetic acid. Mobile phase B is acetonitrile/20mM tributylamine/30mM acetic acid. The solvent flow rate is 1.0 mL/min. The isocratic condition is kept at 26% mobile phase B. The valve, sample loop, and needle are washed with 50% acetonitrile: 50% methanol for 20 seconds. The column temperature is kept at 55°C. Calibration curves are calculated by least-square linear regression with 1/x weighting. 2-HG and α-KG are quantified using standard curve and ratio of the peak area of analytes to internal standard. Data analysis is performed using MultiQuant 3.0 (AB Sciex). The raw data are exported to Excel spreadsheets.

**Determination of IC₅₀ Curves.** IC₅₀ Curves for each compound are obtained using four parameter data fitting analysis in GraphPad/Prism software.

In experiments performed essentially as described above, the IC₅₀ results set forth in Table 1 are obtained.

| **Table 1. IC₅₀ Results** | | |
|---|---|---|
| **Cell Line Construct** | **Ivosidenib IC₅₀ (nM)** | **Compound A IC₅₀ (nM)** |
| U87MG R132H | 27 | 0.32 |
| U87MG R132H_S280F | >1000 | 2.58 |
| U87MG R132C_S280F | >1000 | 12.48 |
| Ba/F3 R132H | 15.92 | 0.11 |
| Ba/F3 R132H_S280F | >1000 | 1.13 |
| Ba/F3 R132C_S280F | >1000 | 1.53 |

The results in Table 1 indicate that each of ivosidenib and Compound A is effective in inhibiting R132H mutant IDH1 in each R132H construct cell line. However, while Compound A is effective in inhibiting R132H_S280F mutant IDH1 and R132C_S280F in each cell line construct, ivosidenib is not effective in inhibiting R132H_S280F mutant IDH1 in either cell line construct and ivosidenib is not effective in inhibiting R132C_S280F mutant IDH1 in either cell line construct.

In experiments performed essentially as described above, the IC50 results set forth in Table 2 are obtained.

| **Table 2. IC50 Results** | | |
|---|---|---|
| Cell Line Construct | Compound A IC₅₀ (nM) | Std Dev |
| | | |
| U87MG IDH1R132H_H315D cis | 1.49 | 0.2 |
| U87MG IDH1R132H_R119P cis | 2.52 | 0.46 |
| U87MG IDH1R132H_G131A cis | 5.89 | 0.88 |
| U87MG IDH1R132H_D279N cis | 13.8 | 2.5 |
| | | |
| U87MG IDH1R132C_H315D cis | 1.33 | 0.42 |
| U87MG IDH1R132C_R119P cis | 10.5 | 3.8 |
| U87MG IDH1R132C_G289D cis | 23.5 | 1.6 |
| U87MG IDH1R132C_G131A cis | 25.6 | 6.5 |
| U87MG IDH1R132C_D279N cis | 101.7 | 13.9 |
| | | |
| U87MG IDH1R132L_H315D cis | 5.07 | 0.5 |
| U87MG IDH1R132L_R119P cis | 89.7 | 31.3 |
| U87MG IDH1R132L_G289D cis | 29.4 | 4.1 |
| U87MG IDH1R132L_G131A cis | 64.5 | 65.2 |
| U87MG IDH1R132L_S280F cis | 179 | 62 |
| U87MG IDH1R132L_D279N cis | 645 | 142 |

The results in Table 2 indicate that Compound A is effective in inhibiting IDH1 second site resistant mutants in the context of IDH1 R132H, R132C or R132L driver mutations.

## Claims

1. A compound of the Formula: 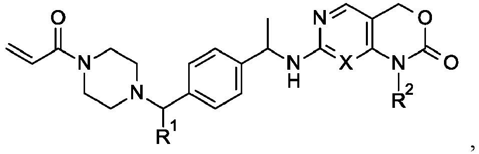 wherein:
R¹ is -CH₂CH(CH₃)₂, -CH₂CH₃, -CH₂CH₂OCH₃, or
-CH₂-cyclopropyl;
R² is -CH₃ or -CH₂CH₃; and
X is N or CH,
or a pharmaceutically acceptable salt thereof;
for use in treating cancer in a human subject having an IDH1 R132 mutation and one or more secondary IDH1 mutations.

2. The compound for use according to claim 1, wherein X is N, or a pharmaceutically acceptable salt thereof.

3. The compound for use according to either claim 1 or 2, wherein R¹ is - CH₂-cyclopropyl, or a pharmaceutically acceptable salt thereof.

4. The compound for use according to any one of claims 1 to 3, wherein R² is -CH₂CH₃, or a pharmaceutically acceptable salt thereof.

5. The compound for use according to any one of claims 1, 2 and 4, wherein the compound is:
7-[[(1S)-1-[4-[(1R)-2-cyclopropyl-1-(4-prop-2-enoylpiperazin-1-yl)ethyl]phenyl]ethyl]amino]-1-ethyl-4H-pyrimido[4,5-d][1,3]oxazin-2-one;
7-[[(1S)-1-[4-[(1S)-2-cyclopropyl-1-(4-prop-2-enoylpiperazin-1-yl)ethyl]phenyl]ethyl]amino]-1-ethyl-4H-pyrimido[4,5-d][1,3]oxazin-2-one; or
1-ethyl-7-[[(1S)-1-[4-[1-(4-prop-2-enoylpiperazin-1-yl)propyl]phenyl]ethyl]amino]-4H-pyrimido[4,5-d][1,3]oxazin-2-one;
or a pharmaceutically acceptable salt thereof.

6. The compound for use according to any one of claims 1 to 5, wherein the compound is: 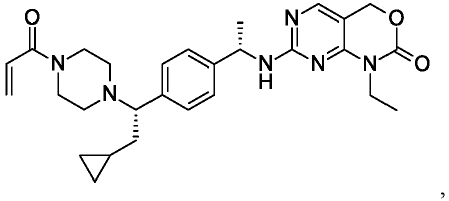 or a pharmaceutically acceptable salt thereof.

7. The compound for use according to claim 6, wherein the compound is:

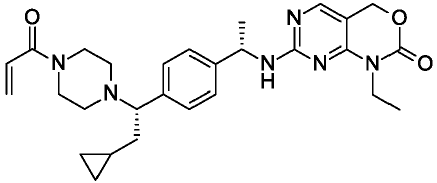

8. The compound for use according to any one of claims 1 to 7, wherein the one or more secondary IDH1 mutations is one or more of R119P, G131A, D279N, S280F, G289D or H315D.

9. The compound for use according to any one of claims 1 to 8, wherein the cancer is a solid tumor cancer.

10. The compound for use according to claim 9, wherein the solid tumor cancer is cholangiocarcinoma, head and neck cancer, chondrosarcoma, hepatocellular carcinoma, melanoma, pancreatic cancer, astrocytoma, oligodendroglioma, glioma, glioblastoma, bladder carcinoma, colorectal cancer, lung cancer, or sinonasal undifferentiated carcinoma.

11. The compound for use according to claim 10, wherein the solid tumor cancer is cholangiocarcinoma.

12. The compound for use according to any one of claims 1 to 8, wherein the cancer is a hematologic malignancy.

13. The compound for use according to claim 12, wherein the hematologic malignancy is acute myeloid leukemia, myelodysplastic syndrome myeloproliferative neoplasm, angioimmunoblastic T-cell lymphoma, T-cell acute lymphoblastic leukemia, polycythemia vera, essential thrombocythemia, primary myelofibrosis, or chronic myelogenous leukemia.

14. The compound for use according to claim 13, wherein the hematologic malignancy is acute myeloid leukemia.

15. The compound for use according to any one of claims 1 to 14, wherein the subject had been treated with a mutant IDH1 inhibitor other than a compound of the formula: 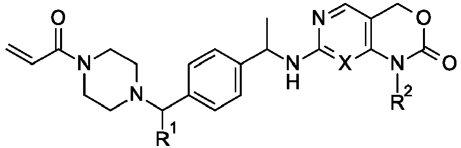 wherein:
R¹ is -CH₂CH(CH₃)₂, -CH₂CH₃, -CH₂CH₂OCH₃, or
-CH₂-cyclopropyl;
R² is -CH₃ or -CH₂CH₃; and
X is N or CH, or a pharmaceutically acceptable salt thereof.

16. The compound for use according to claim 15, wherein the subject had been treated with ivosidenib.

## Patentansprüche

1. Verbindung der Formel: 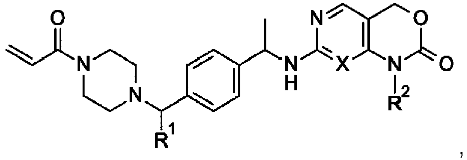 wobei:
R¹ -CH₂CH(CH₃)₂, -CH₂CH₃, -CH₂CH₂OCH₃ oder
-CH₂-Cyclopropyl ist;
R² -CH₃ oder -CH₂CH₃ ist; und
X N oder CH
oder ein pharmazeutisch verträgliches Salz davon ist;
zur Verwendung bei der Behandlung von Krebs in einem menschlichen Subjekt, das eine IDH1-R132-Mutation und eine oder mehrere sekundären IDH1-Mutationen aufweist.

2. Verbindung zur Verwendung nach Anspruch 1, wobei X N oder ein pharmazeutisch verträgliches Salz davon ist.

3. Verbindung zur Verwendung nach Anspruch 1 oder 2, wobei R¹ -CH₂-Cyclopropyl oder ein pharmazeutisch verträgliches Salz davon ist.

4. Verbindung zur Verwendung nach einem der Ansprüche 1 bis 3, wobei R² -CH₂CH₃ oder ein pharmazeutisch verträgliches Salz davon ist.

5. Verbindung zur Verwendung nach einem der Ansprüche 1, 2 und 4, wobei die Verbindung Folgendes ist:
7-[[(1S)-1-[4-[(1R)-2-Cyclopropyl-1-(4-prop-2-enoylpiperazin-1-yl)ethyl]phenyl]ethyl]amino]-1-ethyl-4H-pyrimido[4,5-d][1,3]oxazin-2-on;
7-[[(1S)-1-[4-[(1S)-2-Cyclopropyl-1-(4-prop-2-enoylpiperazin-1-yl)ethyl]phenyl]ethyl]amino]-1-ethyl-4H-pyrimido[4,5-d][1,3]oxazin-2-on; oder
1-Ethyl-7-[[(1S)-1-[4-[1-(4-prop-2-enoylpiperazin-1-yl)propyl]phenyl]ethyl]amino]-4H-pyrimido[4,5-d][1,3]oxazin-2-on; oder ein pharmazeutisch verträgliches Salz davon.

6. Verbindung zur Verwendung nach einem der Ansprüche 1 bis 5, wobei die Verbindung Folgendes ist: 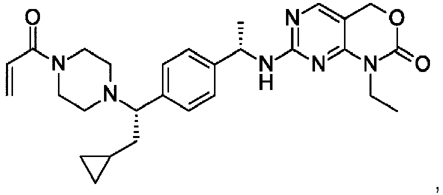 oder ein pharmazeutisch verträgliches Salz davon.

7. Verbindung zur Verwendung nach Anspruch 6, wobei die Verbindung Folgendes ist:

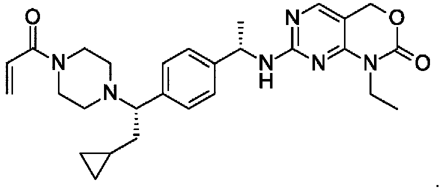

8. Verbindung zur Verwendung nach einem der Ansprüche 1 bis 7, wobei die eine oder die mehreren sekundären IDH1-Mutationen eine oder mehrere von R119P, G131A, D279N, S280F, G289D oder H315D sind.

9. Verbindung zur Verwendung nach einem der Ansprüche 1 bis 8, wobei der Krebs ein solider Tumorkrebs ist.

10. Verbindung zur Verwendung nach Anspruch 9, wobei der feste Tumorkrebs Cholangiokarzinom, Kopf- und Halskrebs, Chondrosarkom, hepatozelluläres Karzinom, Melanom, Bauchspeicheldrüsenkrebs, Astrozytom, Oligodendrogliom, Gliom, Glioblastom, Blasenkarzinom, Kolorektalkrebs, Lungenkrebs oder sinunasales undifferenziertes Karzinom ist.

11. Verbindung zur Verwendung nach Anspruch 10, wobei der solide Tumorkrebs Cholangiokarzinom ist.

12. Verbindung zur Verwendung nach einem der Ansprüche 1 bis 8, wobei der Krebs eine hämatologische Malignität ist.

13. Verbindung zur Verwendung nach Anspruch 12, wobei die hämatologische Malignität akute myeloische Leukämie, myelodysplastisches Syndrom myeloproliferatives Neoplasma, angioimmunoblastisches T-Zell-Lymphom, akute lymphoblastische T-Zell-Leukämie, Polycythaemia vera, essentielle Thrombozythie, primäre Myelofibrose oder chronische myeloische Leukämie ist.

14. Verbindung zur Verwendung nach Anspruch 13, wobei die hämatologische Malignität akute myeloische Leukämie ist.

15. Verbindung zur Verwendung nach einem der Ansprüche 1 bis 14, wobei das Subjekt mit einem anderen mutierten IDH1-Hemmer behandelt wurde als eine Verbindung der Formel: 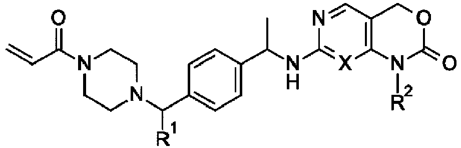 wobei:
R¹ -CH₂CH(CH₃)₂, -CH₂CH₃, -CH₂CH₂OCH₃ oder
-CH₂-Cyclopropyl ist;
R² -CH₃ oder -CH₂CH₃ ist; und
X N oder CH oder ein pharmazeutisch verträgliches Salz davon ist.

16. Verbindung zur Verwendung nach Anspruch 15, wobei das Subjekt mit Ivosidenib behandelt wurde.

## Revendications

1. Composé de la Formule : 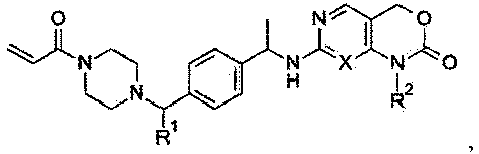 où :
R¹ est -CH₂CH(CH₃)₂, -CH₂CH₃, -CH₂CH₂OCH₃, ou
-CH₂-cyclopropyle ;
R² est -CH₃ ou -CH₂CH₃ ; et
X est N ou CH,
ou un sel pharmaceutiquement acceptable de celui-ci ;
pour une utilisation dans le traitement d'un cancer chez un sujet humain ayant une mutation IDH1 R132 et une ou plusieurs mutations IDH1 secondaires.

2. Composé pour une utilisation selon la revendication 1, où X est N, ou un sel pharmaceutiquement acceptable de celui-ci.

3. Composé pour une utilisation selon la revendication 1 ou 2, où R¹ est - CH₂-cyclopropyle, ou sel pharmaceutiquement acceptable de celui-ci.

4. Composé pour une utilisation selon l'une quelconque des revendications 1 à 3, où R² est -CH₂CH ₃, ou un sel pharmaceutiquement acceptable de celui-ci.

5. Composé pour une utilisation selon l'une quelconque des revendications 1, 2 et 4, où le composé est :
7-[[(1S)-1-[4-[(1R)-2-cyclopropyl-1-(4-prop-2-énoylpipérazin-1-yl)éthyl]phényl]éthyl]amino]-1-éthyl-4H-pyrimido[4,5-d][1,3]oxazin-2-one ;
7-[[(1S)-1-[4-[(1S)-2-cyclopropyl-1 -(4-prop-2-énoylpipérazin-1 - yl)éthyl]phényl]éthyl]amino]-1 -éthyl-4H-pyrimido[4,5-d][1,3]oxazin-2-one ; ou
1-éthyl-7-[[(1S)-1-[4-[1 -(4-prop-2-énoylpipérazin-1 - yl)propyl]phényl]éthyl]amino]-4H-pyrimido[4,5-d][1,3]oxazin-2-one ; ou un sel pharmaceutiquement acceptable de celui-ci.

6. Composé pour une utilisation selon l'une quelconque des revendications 1 à 5, où le composé est : 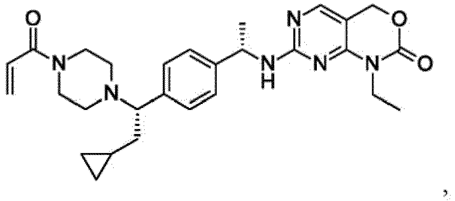 ou un sel pharmaceutiquement acceptable de celui-ci.

7. Composé pour une utilisation selon la revendication 6, où le composé est :

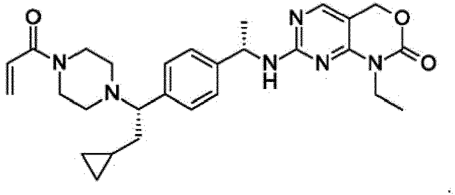

8. Composé pour une utilisation selon l'une quelconque des revendications 1 à 7, où la ou les mutations IDH1 secondaires sont une ou plusieurs parmi R119P, G131A, D279N, S280F, G289D ou H315D.

9. Composé pour une utilisation selon l'une quelconque des revendications 1 à 8, où le cancer est un cancer à tumeur solide.

10. Composé pour une utilisation selon la revendication 9, où le cancer à tumeur solide est un cholangiocarcinome, un cancer de la tête et du cou, un chondrosarcome, un carcinome hépatocellulaire, un mélanome, un cancer du pancréas, un astrocytome, un oligodendrogliome, un gliome, un glioblastome, un carcinome de la vessie, un cancer colorectal, un cancer du poumon, ou un carcinome naso-sinusal indifférencié.

11. Composé pour une utilisation selon la revendication 10, où le cancer à tumeur solide est un cholangiocarcinome.

12. Composé pour une utilisation selon l'une quelconque des revendications 1 à 8, où le cancer est une malignité hématologique.

13. Composé pour une utilisation selon la revendication 12, où la malignité hématologique est une leucémie myéloïde aiguë, un syndrome myélodysplasique un néoplasme myéloprolifératif, un lymphome à lymphocytes T angio-immunoblastique, une leucémie lymphoblastique aiguë à lymphocytes T, une polycytémie vraie, une thrombocytémie essentielle, une myélofibrose primaire, ou une leucémie myéloïde chronique.

14. Composé pour une utilisation selon la revendication 13, où la malignité hématologique est une leucémie myéloïde aiguë.

15. Composé pour une utilisation selon l'une quelconque des revendications 1 à 14, où le sujet a été traité avec un inhibiteur de IDH1 mutant autre qu'un composé de la formule : 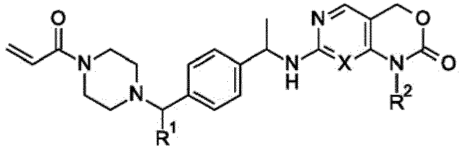 où :
R¹ est -CH₂CH(CH₃)₂, -CH₂CH₃, -CH₂CH₂OCH₃, ou
-CH₂-cyclopropyle ;
R² est -CH₃ ou -CH₂CH₃ ; et
X est N ou CH, ou un sel pharmaceutiquement acceptable de celui-ci.

16. Composé pour une utilisation selon la revendication 15, où le sujet a été traité avec de l'ivosidénib.
